# EUROPEAN PATENT SPECIFICATION

(11) **EP 2 621 877 B1**
(45) Date of publication and mention of the grant of the patent: **25.05.2016**
(21) Application number: 11761593.0
(22) Date of filing: 28.09.2011
(51) Int. Cl.: C07C 29/10, C07C 29/17, C07C 35/50, C07D 249/08

(54) **PROCESS FOR PREPARING 5-[1-(4-CHLOROPHENYL)-METHYLENE]-1-HYDROXYMETHYL-2,2-DIMETHYL-CYCLOPENTANOL**
VERFAHREN ZUR HERSTELLUNG VON 5-[1-(4-CHLORPHENYL)-METHYLEN]-1-HYDROXYMETHYL-2,2-DIMETHYL-CYCLOPENTANOL
PROCÉDÉ DE PRÉPARATION DU 5-[1-(4-CHLOROPHÉNYL)-MÉTHYLÈNE]-1-HYDROXYMÉTHYL-2,2-DIMÉTHYL-CYCLOPENTANOL

(30) Priority: 01.10.2010 EP 10186048
(43) Date of publication of application: 07.08.2013
(73) Proprietor: BASF SE, 67056 Ludwigshafen am Rhein (DE)
(72) Inventor: ZIERKE, Thomas, 67459 Böhl-Iggelheim (DE)
(74) Representative: BASF IP Association
(86) International application number: PCT/EP2011/066803
(87) International publication number: WO 2012/041871

(56) References cited:
- EP-A2- 0 357 404
- US-A- 5 256 683

## Description

The present invention relates to a process for preparing diols of the formula (Ia) and (Ib)

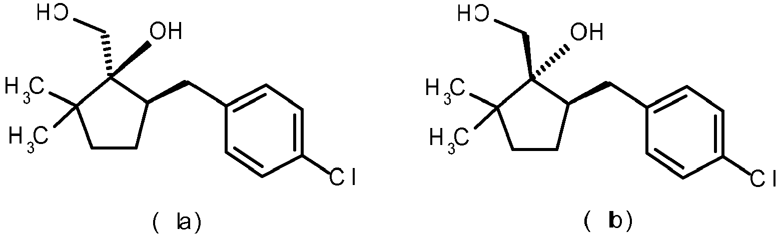

According to EP-A 359 305, compound (Ia) can be obtained by reacting either the corresponding epoxide (VII) or ester (VIII) 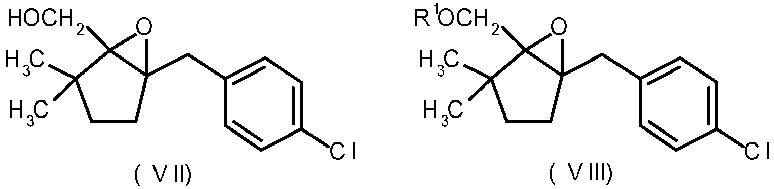 wherein R¹ represents a hydrogen atom, an alkyl group or a cycloalkyl group, with a reducing agent, preferably a complex metal hydride, at a temperature from 35°C to reflux temperature. However, these reagents suffer from being hazardous and expensive. EP-A 359 305 also teaches the use of the diols (Ia) and (Ib) for preparing fungicidal active cyclopentane derivatives such as Metconazole.

EP-A 474303 discloses a method of selectively preparing the diol (Ia) from the well obtainable 1-(4-chlorobenzyl)-4,4-dimethyl-cyclohex-1-en-3-on (IX)

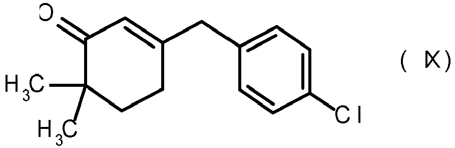

However, this process requires, besides complex metal hydrides, the use of hydrogen per oxide, which requires challenging safety means in a technical plant.

Therefore, it was an object of the present invention to provide a commercial feasible process for preparing the diols of the formula (I) from readily available starting materials, with (Ia) being obtained in an excess relative to the diol (Ib).

It has been found that the diols (Ia) and (Ib) can be prepared in a commercial feasible way by
a) the reaction of an oxirane (II) 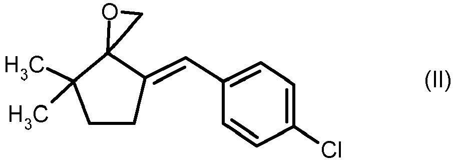 with water and
b) hydrogenation of the resulting 5-[1-(4-chlorophenyl)-meth-(E)-ylidene]-1-hydroxymethyl-2,2-dimethyl-cyclopentanol (III) 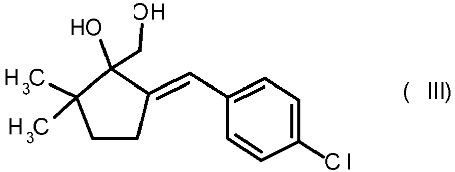 with hydrogen in the presence of a metal catalyst.

The reaction of compound (II) with water is normally carried out at a temperature of (-10) to 40°C, preferably at 5 to 35°C.

The water is used in at least stoichiometric amount or in a superstoichiometric amount with water being a part of the used solvent mixture.

Accordingly, it is expedient to effect the addition of water in a solvent, which preferably is miscible with water. Suitable in this respect are alcohols such as methanol, ethanol, n-propanol, isopropanol, n-butanol, isobutanol and tert.-butanol, ketones such as aceton and methylethylketon, ether such as tetrahydrofuran, dioxan and dimethoxyethan, amides such as dimethylformamid and dimethylacetamid, as well as N-methylpyrrolidon, acetonitril, pyridine or dimethylsulfoxide.

It is also possible to use a co-solvent with a poor solubility for water, to facilitate the work-up of the resulting 5-[1-(4-chlorophenyl)-meth-(E)-ylidene]-1-hydroxymethyl-2,2-dimethyl-cyclopentanol (III). Said co-solvent may be the solvent in which compound (II) was synthesized. In the latter case, the solvent miscible with water is added as a mixture with water to compound (II) without isolating it from the solution, leading to a 2-phase reaction mixture.

In order to speed up the reaction time it may be advantageous to add trace amounts of an acid. Suitable acids are mineral acids such as hydrochloric acid, sulfuric acid or phosphoric acid, boric acid, sulfonic acids such as methan, dodecyl, benzene, dodecylbenzene, or p-toluenesulfonic acid, carboxylic acids such as formic acid, acetic acid, benzoic acid optionally substituted by a chlorine, fluorine, bromine atom or a nitro group, trichloracetic acid or trifluoroacetic acid, or salts of these acids with amines such as ammoniac, triethylamin, dimethylamin, diethylamin, dimethylcyclohexylamin, diisopropylethylamin and tri-n-butylamin, acid tetraalkyl ammonium salts such as tetrabutylammoniumhydrogensulfate, and acid buffer systems containing alkali metal salts of sulfuric acid, phosphoric acid, formic acid, acetic acid and the respective acids.

The hydrogenation of the 5-[1-(4-chlorophenyl)-meth-(E)-ylidene]-1-hydroxymethyl-2,2-dimethyl-cyclopentanol (III) is normally effected in a hydrogen atmosphere at a temperature of (-10) to 40°C, preferably at 0 to 30°C.

The hydrogen pressure can be varied in a wide range, suitably between 1 and 100 bar, preferably within a range from 5 to 80 bar.
Generally, pressures above 1 bar may positively influence the uptake of hydrogen.

Suitable metal catalysts are iridium, rhodium, ruthenium, palladium, platinum and nickel either in heterogeneous form with great specific surface or on a support material such as charcoal, aluminium oxide or silica gel. However, it is also possible to use salts of the above metals, optionally modified with complex ligands such as alkyl or aryl phosphines to become soluble in the reaction mixture.

Typically, the hydrogenation is effected in an alcohol such as methanol, ethanol and isopropanol, an ether such as diethylether, methyl-tert.-butylether, tetrahydrofurane, 2-methyl-tetrahydrofurane, 3-methyl-tetrahydrofurane, dioxane and dimethoxyethane, an aliphatic hydrocarbon such as hexane, heptane and cyclohexane, a keton such as acetone, cyclohexanone and methylethylketone, an aromatic hydrocarbon such as benzene, toluene and the xylenes, a carboxylic ester such as acetic acid ethyl ester or butyl acetate, a dipolar aprotic solvent such as di(C₁-C₄-alkyl)formamids (especially dimethylformamide), di(C₁-C₄-alkyl)acetamids (especially dimethylacetamide) and N-methyl-2-pyrrolidone.

According to this invention, the particular combination of catalyst and solvent or solvent mixture is critical to the formation of (Ia) and/or (Ib).

The diols (Ia) and (Ib) are valuable intermediates for the manufacture of Metconazole [CAS No. 125116-23-6; IUPAC name: (1RS,5RS,1RS,5SR)-5-(4-chlorobenzyl)-2,2-dimethyl-1-(1H-1,2,4-triazol-1ylmethyl)cyclopentanol], consisting of a diastereomeric mixture of isomer (Xa) in at least 80 mol% and isomer (Xb) in up to 20 mol% (see EC review report for the active substance metconazole Sanco/10027/2006, Appendix 1, p.1):

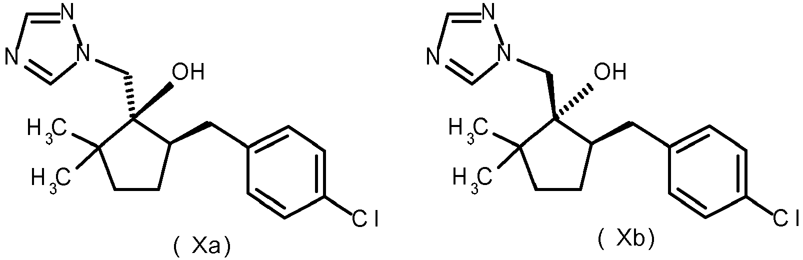

Accordingly, in case the diols (Ia) and (Ib) are intended to be further processed to Metconazole, it is desirous to choose catalyst and solvent or solvent mixture such that the obtained mixture of (Ia) and (Ib) contains at least 80mol% of (Ia) relative to (Ib). For example, processing the hydrogenation with a palladium catalyst in dimethylformamide, dimethylacetamide or N-methyl-2-pyrrolidone has proven to be suitable.

The diols (Ia) and (Ib) can be converted according to EP-A 359305 and EP-A 357404 into the corresponding compounds (IVa) and (IVb) 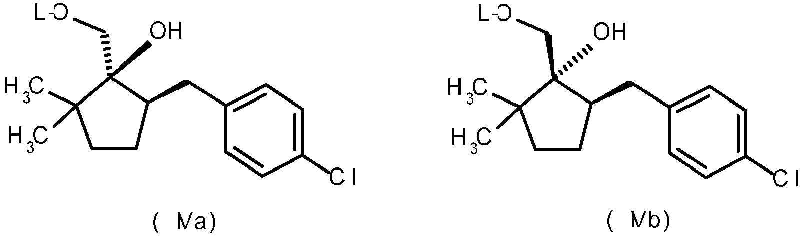 in which L is an optionally substituted alkylsulfonyl or arylsulfonyl group, preferably C₁-C₆-(halo)-alkylsulfonyl such as methylsulfonyl or trifluormethylsulfonyl and unsubstituted or with halogen, C₁-C₄-alkyl or nitro substituted phenylsulfonyl such as 4-methylphenylsulfonyl, 4-chlorophenylsulfonyl or nitrophenylsulfonyl.

Subsequent reaction of (IVa) and (IVb) with a triazole (V) 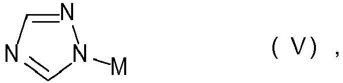 in which M is an alkali metal, preferably sodium or potassium, in the presence of a base, or conversion of (IVa) and (IVb) in the presence of a base into the epoxides (VIa) and (VIb) 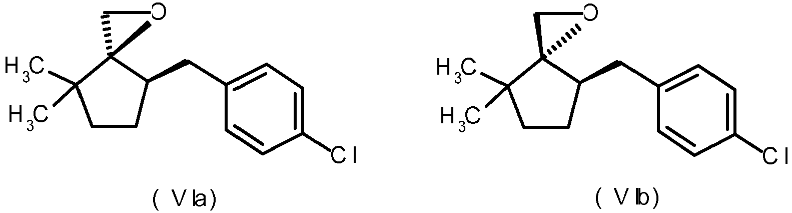 and subsequent reaction of (VIa) and (Vlb) with a triazole (V) leads to Metconazole.

Generally, an approximately stoichiometric amount of a base is used, based on (IVa) and (IVb). The base can, however, also be used in a superstoichiometric amount. In general, the base is used in an amount of from 0.5 to 10 mol and especially in the amount of from 0.9 to 5 mol per mol (IVa)/(IVb). Preference is given to working with an amount of from 1 to 3 mol per mol (IVa)/(IVb).

Suitable bases are organic and inorganic bases.

Suitable inorganic bases are, for example, alkali metal and alkaline earth metal hydroxides, such as lithium hydroxide, sodium hydroxide, potassium hydroxide and calcium hydroxide, alkali metal and alkaline earth metal oxides, such as lithium oxide, sodium oxide, calcium oxide and magnesium oxide, alkali metal and alkaline earth metal hydrides, such as lithium hydride, sodium hydride, potassium hydride and calcium hydride, alkali metal and alkaline earth metal carbonates, such as lithium carbonate, potassium carbonate and calcium carbonate, and also alkali metal bicarbonates, such as sodium bicarbonate. Preference is given to an aqueous NaOH solution or an aqueous KOH solution.
The organic base advantageously is an amine base, i.e. a base wherein the site of basicity is a nitrogen atom. Preferably, the amine base is a tertiary alkyl-, alkenyl-, or alkinylamine or an arylamine or a heterocyclic aromatic amine. Preference is given to triethylamin, dimethylcyclohexylamine, diisopropylethylamine and tri-n-butylamine, N-methyl pyrrolidine, N-methyl piperidine, N-methyl morpholine, N,N'-dimethyl piperazine, DABCO (1,4-diazabicyclo[2.2.2]octane), DBU (1,8-diazabicyclo[5.4.0]undec-7-ene), DBN (1,5-diazabicyclo[4.3.0]non-5-ene), DBU (1,8-diazabicyclo[5.4.0]undec-7-ene) and DBN (1,5-diazabicyclo[4.3.0]non-5-ene), pyridine, 2-picoline, 3-picoline, 4-picoline, 5-ethyl-2-methylpyridine, 2-ethylpyridine, 2,3-lutidine, 2,4-lutidine, 2,5-lutidine, 2,6-lutidine, 3,4-lutidine, 3,5-lutidine, 2,4,6-collidine, 2,3,5-collidine.

Typically, the conversion of (IVa)/(IVb) or of the epoxides (VIa)/(VIb) into Metconazole is effected in an inert dipolar aprotic organic solvent. Examples of such solvents are nitriles such as acetonitrile and propionitrile, dimethylformamide, dimethyl-acetamide, N-methylpyrrolidone, di-methylsulfoxide mixtures thereof. The preference is given to dimethylformamide and N-methylpyrrolidone.

The starting oxirane (II) can be obtained from adipinic acid dimethylester according to EP-A 751111 via the ketone (XI) 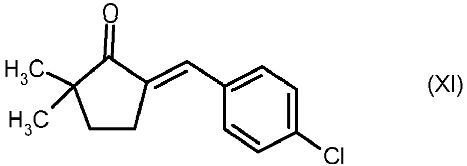 and subsequent conversion with dimethylsulfoniummethylid according to EP-A 467792. Dimethylsulfoniummethylid is obtainable from trimethylsulfonium salts in the presence of a base according to E.J. Corey, M. Chaykovsky, JACS 87, 1965, p. 1353ff.
The preparation examples which follow serve to further illustrate the invention.

### Example 1: Preparation of E-5-[1-(4-Chlorobenzyliden]-1-hydroxymethyl-2,2-dimethyl-cyclopentanol (Formula III)

50 ml dimethylformamide were added to 181 g of a 24 wt-% solution of 7-[1-(4-Chloro-phenyl)-meth-(E)-ylidene]-4,4-dimethyl-1-oxa-spiro[2.4]heptane (Formula II) in toluene. Then 110 ml of a 0,0025 molar hydrochloric acid were added such, that the temperature remained between a range of 23 to 27°C. The two-phasic reaction mixture was stirred for 4 h between 20 to 25°C. Then the aqueous phase was discarded. The toluene phase was extracted once with 140 ml of water and then evaporated at 35°C/6mbar. The residue was treated with 120 ml n-pentane for crystallization. The solid was filtered off, washed with 30 ml of fresh n-pentane and dried on the filter in a stream of N₂-gas. Thus 32,6 g of slightly yellow solid were obtained.
Melting point: 84 - 85°C;
TOF-MS: m/e ES+ 333,1287 (M + Acetonitril + Na); ES- 311,1072 (M + Formiat) Elemental analysis: C 67,6%, H 7,1%, O 12,5%, Cl 13,2%
¹H-NMR (500 MHz, DMSO-d₆): δ/ppm = 0,85 (s, 3H), 0,98 (s, 3H), 1,52-1,61 (m, 1 H), 1,66-1,72 (m, 1 H), 2,50 - 2,55 (m, 2H), 3,44 - 3,52 (m, 2H), 4,38 (s, OH), 4,40 - 4,45 (t, CH₂OH), 6,46 (s, 1 H), 7,35 - 7,41 (m, 4 H);
¹³C-NMR (125 MHz, DMSO-d₆): δ/ppm = 22,07 (q), 24,10 (q), 26,54 (t), 36,09 (t), 42,1 (s), 65,54 (t), 83,28 (s), 121,31 (d), 128,21 (d, 2C), 129,85 (d, 2C), 130,32 (s), 136,80 (s), 150,00 (s).

### Example 2a: Synthesis of (1-RS,5SR)-5-(4-Chloro-benzyl)-1-hydroxymethyl-2,2-dimethyl-cyclopentanol (Formula Ia) and (1RS,5RS)-5-(4-Chloro-benzyl)-1-hydroxymethyl-2,2-dimethyl-cyclopentanol (Formula Ib)

4 g E-5-[1-(4-Chlorobenzyliden]-1-hydroxymethyl-2,2-dimethyl-cyclopentanol (Formula III), purity 92,4 wt-%, dissolved in 22 g of a solvent mixture containing 9 parts toluene and 1 part dimethylformamide (wt/wt) were transferred to pressurized vessel. 0,2 g Pd/C (5-wt-%) were added. The vessel was closed and the mixture was stirred under 80 bar hydrogen pressure for 6 h at 25°C. After separation from the catalyst by filtration, the product solution was evaporated at 80°C/1mbar. The residue of 4,5 g contained 61,5 wt-% of compound (Ia) and 14,0 wt-% of compound (Ib), determined by quantitative HPLC-assay. Total yield for both isomers: 91,3%.

The single isomers have been isolated by H PLC column chromatography for the purpose of characterization.
(1 RS,5SR)-5-(4-Chloro-benzyl)-1-hydroxymethyl-2,2-dimethyl-cyclopentanol (Formula Ia); Melting point: 106°C;
¹H-NMR (400 MHz, CDCl₃): δ/ppm = 0,94 (s, 3H), 1,08 (s, 3H), 1,26-1,48 (m, 2H), 1,58-1,81 (m, 2H), 1,81 - 2,08 (s, broad, 2 OH), 2,16 - 2,28 (m, 1H), 2,44-2,57 (dd, 1 H), 2,90-3,0 (dd, 1 H), 3,59- - 3,64 (d, 1 H), 3,66- - 3,75 (d, 1 H), 7,08- 7,17 (d, 2 H), 7,20- 7,29 (d, 2 H);
¹³C-NMR (125 MHz, CDCl₃): δ/ppm = 23,27 (q), 25,46(q), 27,32 (t), 36,08 (t), 38,25 (t), 45,68 (s), 46,63 (d), 65,65 (t), 128,26 (d, 2C), 130,14 (d, 2C), 131,28 (s), 140,33 (s) (1RS,5RS)-5-(4-Chloro-benzyl)-1-hydroxymethyl-2,2-dimethyl-cyclopentanol (Formula Ib); Melting point: 79°C;
¹H-NMR (400 MHz, CDCl₃): δ/ppm = 0,98 (s, 3H), 1,04 (s, 3H), 1,21-1,35 (m, 1 H), 1,39-1,47 (m, 1 H), 1,60 - 1,73 (m, 2H), 1,79 - 2,06 (s, broad 2 OH), 2,27- 2,39 (m, 2H), 3,13-3,19 d, 1H), 3,68-3,78 (dd, 2H), 7,08 - 7,29 (m, 4 H);
¹³C-NMR (125 MHz, CDCl₃): δ/ppm = 23,74 (q), 24,37 (q), 26,97 (t), 37,97 (t), 38,02 (t), 44,33 (s), 50,43 (d), 62,94 (t), 128,26 (d, 2C), 130,00 (d, 2C), 131,37 (s), 140,06 (s).

### Example 2b: Synthesis of (1-RS,5SR)-5-(4-Chloro-benzyl)-1-hydroxymethyl-2,2-dimethyl-cyclopentanol (Formula Ia) and (1RS,5RS)-5-(4-Chloro-benzyl)-1-hydroxymethyl-2,2-dimethyl-cyclopentanol (Formula Ib)

4 g E-5-[1-(4-Chlorobenzyliden]-1-hydroxymethyl-2,2-dimethyl-cyclopentanol (Formula III), purity 99,1 wt-%, dissolved in 22 g dimethylformamide, were transferred to a pressurized vessel. 0,2 g Pd/C (5-wt-%) were added. The vessel was closed and the mixture was stirred under 80 bar hydrogen pressure for 6 h at 25°C. After separation from the catalyst by filtration, the product solution was evaporated at 80°C/1 mbar. The residue of 4,6 g contained 56,8 wt-% of compound (Ia) and 10,8 wt-% of compound (Ib), determined by quantitative HPLC-assay. Total yield for both isomers: 77,8%.
The single isomers have been isolated by HPLC column chromatography for the purpose of characterization.

### Example 2c: Synthesis of (1 RS,5SR)-5-(4-Chloro-benzyl)-1-hydroxymethyl-2,2-dimethyl-cyclopentanol (Formula Ia) and (1RS,5RS)-5-(4-Chloro-benzyl)-1-hydroxymethyl-2,2-dimethyl-cyclopentanol (Formula Ib)

4 g E-5-[1-(4-Chlorobenzyliden]-1-hydroxymethyl-2,2-dimethyl-cyclopentanol (Formula III), purity 99,1 wt-%, dissolved in 22 g dimethylacetamide, were transferred to a pressurized vessel. 0,2 g Pd/C (5-wt-%) were added. The vessel was closed and the mixture was stirred under 80 bar hydrogen pressure for 6 h at 25°C. After separation from the catalyst by filtration the product solution was evaporated at 80°C/1 mbar. The residue of 6,6 g contained 40,6 wt-% of compound (Ia) and 6,8 wt-% of compound (Ib), determined by quantitative HPLC-assay. Total yield for both isomers: 84,0%.

### Example 2d: Synthesis of (1RS,5SR)-5-(4-Chloro-benzyl)-1-hydroxymethyl-2,2-dimethyl-cyclopentanol (Formula la) and (1RS,5RS)-5-(4-Chloro-benzyl)-1-hydroxymethyl-2,2-dimethyl-cyclopentanol (Formula Ib)

2 g E-5-[1-(4-Chlorobenzyliden]-1-hydroxymethyl-2,2-dimethyl-cyclopentanol (Formula VIII), purity 99,0 wt-%, dissolved in 43,6 g toluene, were transferred to pressurized vessel. 0,4 g Pd/C (5-wt-%) were added. The vessel was closed and the mixture was stirred under 80 bar hydrogen pressure for 21 h at 25°C. After separation from the catalyst by filtration the product solution was analyzed by HPLC. The solution contained the diols of formula (Ia) and (Ib) in a ratio of 2,2 : 1.

### Example 2e: Synthesis of (1RS,5SR)-5-(4-Chloro-benzyl)-1-hydroxymethyl-2,2-dimethyl cyclopentanol (Formula Ia) and (1RS,5RS)-5-(4-Chloro-benzyl)-1-hydroxymethyl-2,2-dimethyl-cyclopentanol (Formula Ib)

2 g E-5-[1-(4-Chlorobenzyliden]-1-hydroxymethyl-2,2-dimethyl-cyclopentanol (Formula III), purity 99,0 wt-%, dissolved in 43,6 g tetrahydrofurane, were transferred to a pressurized vessel. 0,4 g Pd/C (5-wt-%) were added. The vessel was closed and the mixture was stirred under 80 bar hydrogen pressure for 22 h at 25°C. After separation from the catalyst by filtration the product solution was evaporated at 80°C/1 mbar. The residue of 1,7 g contained the diols of formula (Ia) and (Ib) in a ratio of 3,8 : 1 (HPLC assay).

### Example 2f: Synthesis of (1RS,5SR)-5-(4-Chloro-benzyl)-1-hydroxymethyl-2,2-dimethyl cyclopentanol (Formula Ia) and (1RS,5RS)-5-(4-Chloro-benzyl)-1-hydroxymethyl-2,2-dimethyl-cyclopentanol (Formula Ib)

2 g E-5-[1-(4-Chlorobenzyliden]-1-hydroxymethyl-2,2-dimethyl-cyclopentanol (Formula III), purity 99,0 wt-%, dissolved in 31,4 g Isopropanol, were transferred to pressurized vessel. 0,1 g Pd/C (5-wt-%) were added. The vessel was closed and the mixture was stirred under 80 bar hydrogen pressure for 1 h at 25°C. After separation from the catalyst by filtration the product solution was evaporated at 80°C/1mbar. The residue of 2,1 g contained the diols (Ia) and (Ib) in a ratio of 2,4 : 1 (HPLC assay).

### Example 3: Synthesis of Methanesulfonic acid (1RS,5SR)-5-(4-chlorobenzyl)-1-hydroxy-2,2-dimethyl-cyclopentylmethyl ester (Formula (IVa), L = Methylsulfonyl) and Methanesulfonic acid (1 RS,5RS)-5-(4-chlorobenzyl)-1-hydroxy-2,2-dimethyl-cyclopentylmethyl ester (Formula (IVb), L = Methylsulfonyl)

3,1 g of Methanesulfonic acid chloride were dosed as a solution in toluene (ca. 50 wt-%) to a pre-charged solution of 6,5 g of a mixture of compounds of Formula (Ia) and (Ib) with a ratio of 6,8 : 1 in favor of compound (Ia) (purity: 95,5 wt-%), 3,8 g of Dimethylcyclohexylamine in 23 g of toluene at 25°C. After dosage the mixture was stirred at 25°C for 1 h. Then 30 g water were added and the mixture was heated to 60°C. After phase separation the toluene phase was washed again with 30 g water. Then the toluene was stripped off at 60°C / 1 mbar. 7,8 g of a white solid were obtained as residue. The solid contained 77,4 wt-% of Methanesulfonic acid (1 RS,5SR)-5-(4-chlorobenzyl)-1-hydroxy-2,2-dimethyl-cyclopentylmethyl ester (Formula IVa, L = Methylsulfonyl) and 13,1 wt-% of Methanesulfonic acid (1 RS,5RS)-5-(4-chlorobenzyl)-1-hydroxy-2,2-dimethyl-cyclopentylmethyl ester (Formula IVb, L = Methylsulfonyl)
The single isomers have been isolated by H PLC column chromatography for the purpose of characterization.
Methanesulfonic acid (1 RS,5SR)-5-(4-chlorobenzyl)-1-hydroxy-2,2-dimethyl-cyclopentylmethyl ester (Formula IVa, L = Methylsulfonyl):
¹H-NMR (400 MHz, d₆-DMSO): δ/ppm = 0,95 (s, 3H), 1,03 (s, 3H), 2,79-2,84 (dd, 1 H), 3,20 (s,3H);
¹³C-NMR (125 MHz, d₆-DMSO): δ/ppm = 23,24 (q), 25,30 (q), 26,62 (t), 35,33 (t), 36,39 (q), 37,86 (t), 45,29 (d), 45,84 (s), 73,25 (t), 80,55 (s), 127,95 (d, 2C), 130,08 (s), 130,49(d, 2C), 140,62 (s);
Methanesulfonic acid (1RS,5RS)-5-(4-chlorobenzyl)-1-hydroxy-2,2-dimethyl-cyclopentylmethyl ester (Formula IVb, L = Methylsulfonyl):
¹H-NMR (400 MHz, d₆-DMSO): δ/ppm = 0,97 (s, 3H), 0,99 (s, 3H), 2,97-3,03 (dd, 1 H), 3,24 (s,3H);
¹³C-NMR (125 MHz, d₆-DMSO): δ/ppm = 23,26 (q), 25,32 (q), 25,94 (t), 36,03 (t), 36,45 (q), 37,02 (t), 44,10 (s), 49,48 (d), 70,97 (t), 80,30 (s), 128,03 (d, 2C), 130,16 (s), 130,29(d, 2C), 140,48 (s).

### Example 4: Synthesis of (3RS,7SR)-7-(4-Chlorobenzyl)-4,4-dimethyl-1-oxa-spiro[2.4]heptane (Formula VIa) and (3RS,7RS)-7-(4-Chlorobenzyl)-4,4-dimethyl-1-oxa-spiro[2.4]heptane (Formula Vlb)

5,7 g of methanesulfonic acid chloride were dosed as a solution in toluene (ca. 50 wt-%) to a pre-charged solution of 12,5 g of a mixture of compounds of Formula (Ia) and (Ib) with a ratio of 5,6 : 1 in favor of compound (Ia) (purity: 95,5 wt-%), 6,9 g of dimethylcyclohexylamine in 21 g of toluene at 40 -45°C. After dosage the mixture was kept at 50°C for 30 min. Then 35,5 g of a 15 wt-% solution of sodium hydroxide in water were added in a manner that the reaction temperature was kept higher than 45°C. After dosage the mixture was kept at 50°C for 3 h. After phase separation the organic phase was extracted with hydrochloric acid at pH 2-3. After the acidic extraction the organic phase was washed with water until the water phase remained neutral.
Then the organic phase was evaporated at 50°C/ 1mbar. The obtained residue contained an oily mixture of (3RS,7SR)-7-(4-Chlorobenzyl)-4,4-dimethyl-1-oxa-spiro[2.4]heptane (Formula Via) and (3RS,7RS)-7-(4-Chlorobenzyl)-4,4-dimethyl-1-oxa-spiro[2.4]heptane (Formula VIb), in a ratio of 5,9 with a purity of 92,2 wt-%. Thus a total yield of 93,4 % was obtained.
The single isomers have been isolated by HPLC column chromatography for the purpose of characterization.
(3RS,7SR)-7-(4-Chlorobenzyl)-4,4-dimethyl-1-oxa-spiro[2.4]heptane (Formula Via):
¹H-NMR (400 MHz, CDCl₃): δ/ppm = 0,86 (s, 3H), 0,97 (s, 3H), 1,42-1,52 (m, 2H), 1,60-1,70 (m, 1H), 1,70-1,79 (m, 1H), 2,31-2,40 (m, 1H), 2,44-2,54 (m, 2H), 2,56-2,58 (d, 1H), 2,67-2,69 (d, 1 H), 7,08-7,11 (d, 2H), 7,20-7,23 (d, 2H);
¹³C-NMR (125 MHz, CDCl₃): δ/ppm = 22,92 (q), 27,16 (q), 28,41 (t), 35,04 (t), 38,54 (t), 39,63 (s), 42,24 (d), 47,26 (t), 70,29 (s), 128,30 (d, 2C), 130,16 (d, 2C), 131,44 (s), 139,75 (s);
(3RS,7RS)-7-(4-Chlorobenzyl)-4,4-dimethyl-1-oxa-spiro[2.4]heptane (Formula VIb): ¹H-NMR (400 MHz, CDCl₃): δ/ppm = 0,89 (s, 3H), 0,95 (s, 3H), 1,32-1,77 (m, 4H), 2,29-2,39 (m, 1 H), 2,67 (d, 1 H), 2,70-2,78 (m, 1 H), 2,88 (d, 1 H), 7,10 (d, 2H), 7,24 (d, 2H);
¹³C-NMR (125 MHz, CDCl₃): δ/ppm = 23,87 (q), 26,16 (q), 27,56 (t), 38,20 (t), 38,79 (t), 38,97 (s), 44,40 (d), 47,67 (t), 71,49 (s), 128,32 (d, 2C), 130,12 (d, 2C), 131,58 (s), 139,02 (s).

### Example 5: Synthesis of Metconazole, (1RS,5RS,1RS,5SR)-5-(4-chlorobenzyl)-2,2-dimethyl-1-(1H-1,2,4-triazol-1ylmethyl)cyclopentanol (Formula Xa and Xb)

A mixture of (3RS,7SR)-7-(4-Chlorobenzyl)-4,4-dimethyl-1-oxa-spiro[2.4]heptane (Formula VIa) and (3RS,7RS)-7-(4-Chlorobenzyl)-4,4-dimethyl-1-oxa-spiro[2.4]heptane (Formula VIb) in a ratio of 5,9 : 1 with a purity of 93,9 wt-% was dosed to pre-charged 4,3 g of sodium salt of 1,2,4-triazole in dimethylformamide at 90°C. After dosage the mixture was stirred at 90°C for 6 h. Then most of the dimethylformamide was stripped off at 85°C/1mbar. The residue was dissolved between 27 g water and 55 g toluene at 85°C. After phase separation the toluene phase was extracted once again with 24 g water at 85°C. After phase separation the toluene was stripped off at 85°C/1 mbar.
The residue was dissolved in 85 g methylcyclohexane at 75°C. After cooling down to 25°C overnight the suspension of the precipitated product was further cooled to 5°C, filtered and washed two times with 10 g cold methylcyclohexane. The solid product was dried at 50°C/8mbar for 12 h. Thus 9,9 g of Metconazole with a purity of 97,7% as a mixture of isomers of Formula Xa and Xb with a ratio of 7,1 : 1 was obtained, corresponding to a total yield of 77%.

## Claims

1. A process for preparing diols of the formula (Ia) and (Ib) 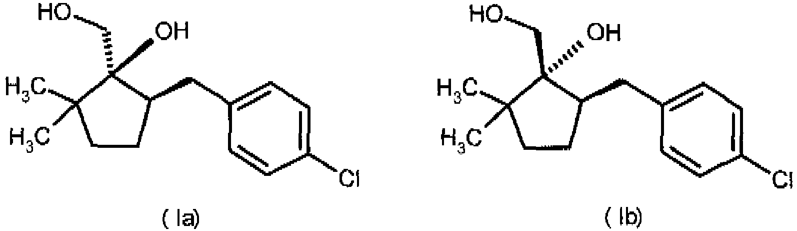 comprising a) the reaction of an oxirane (II) 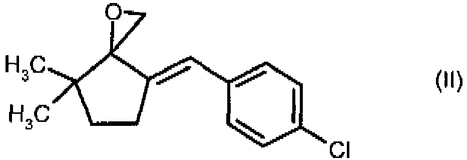 with water and b) hydrogenation of the resulting 5-[1-(4-chlorophenyl)-meth-(E)-ylidene]-1-hydroxymethyl-2,2-dimethyl-cyclopentanol (III) 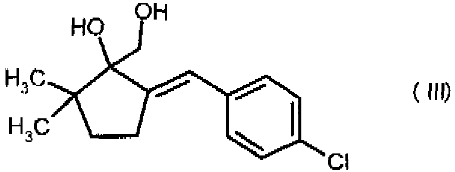 with hydrogen in the presence of a metal catalyst.

2. The process according to claim 1, wherein the reaction a) is carried out at a temperature of (-10) to 40°C.

3. The process according to claim 1, wherein the hydrogenation b) is carried out at a temperature of (-10) to 40°C.

4. The process according to claims to 3, wherein the reaction is carried out in a solvent or solvent mixture,

5. The process according to claim 4, wherein catalyst, temperature and solvent or solvent mixture is selected such that the diol (Ia) is obtained in an excess relative to the dial (Ib).

6. The process according to claim 4, wherein the hydrogenation is carried out in the presence of a di(C₁-C₄-alkyl)formamid, di(C₁-C₄-alkyl)acetamid or in N-methyl-2-pyrrolidone and palladium is used as catalyst.

7. A process according to claims 1 to 6, comprising additionally the subsequent conversion of diols (Ia) and (Ib) into the corresponding compounds (IVa) and (IVb) 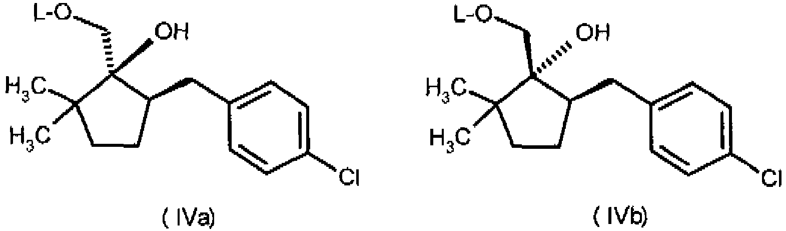 in which L is C₁-C₆-alkylsultonyl, C₁-C₆-haloalkylsulfonyl, phenylsulfonyl or with halogen, C₁-C₄-alkyl or nitro substituted phenylsulfonyl, and either
a) subsequent reaction of (IVa) and (IVb) with a triazole (V) 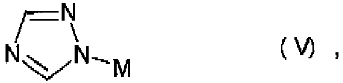 in which M is an alkali metal, in the presence of a base, or
b) conversion of (IVa) and (IVb) in the presence of a base into the epoxides (Via) and (Vlb) 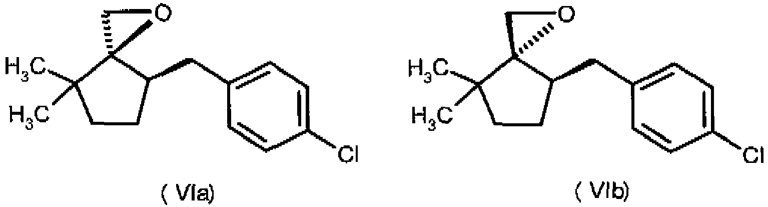 and subsequent reaction of (VIa) and (VIb) with a triazole (V), to obtain Metconazole (Xa) / (Xb)

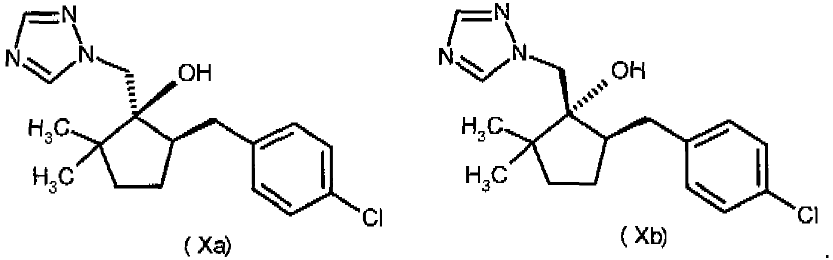

8. 5-[1-(4-chlorophenyl)-meth(E)-ylidene]-1-hydroxymethyl-2,2-dimethyl-cyclopentanol of the formula (III)

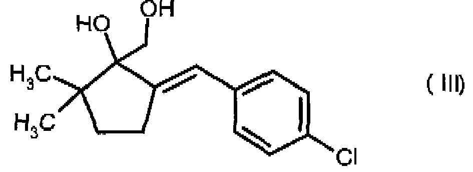

## Patentansprüche

1. Verfahren zur Herstellung von Diolen der Formel (Ia) und (Ib) 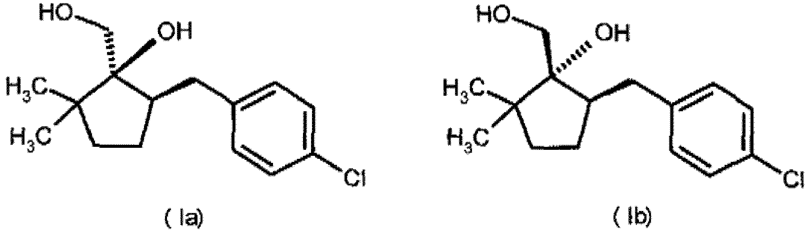 bei dem man a) ein Oxiran (II) 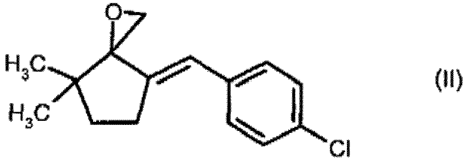 mit Wasser umsetzt und b) das erhaltene 5-[1-(4-Chlorphenyl)meth-(E)-yliden]-1-hydroxymethyl-2,2-dimethylcyclopentanol (III) 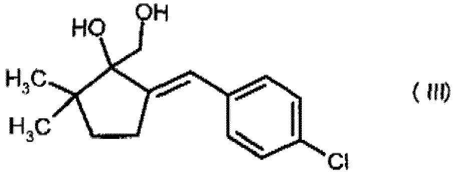 in Gegenwart eines Metallkatalysators mit Wasserstoff hydriert.

2. Verfahren nach Anspruch 1, bei dem die Umsetzung a) bei einer Temperatur von (-10) bis 40°C durchgeführt wird.

3. Verfahren nach Anspruch 1, bei dem die Hydrierung b) bei einer Temperatur von (-10) bis 40°C durchgeführt wird.

4. Verfahren nach einem der Ansprüche 1 bis 3, bei dem die Umsetzung in einem Lösungsmittel oder Lösungsmittelgemisch durchgeführt wird.

5. Verfahren nach Anspruch 4, bei dem Katalysator, Temperatur und Lösungsmittel bzw. Lösungsmittelgemisch so gewählt werden, dass das Diol (Ia) in einem Überschuss im Verhältnis zu dem Diol (Ib) erhalten wird.

6. Verfahren nach Anspruch 4, bei dem die Hydrierung in Gegenwart eines Di(C₁-C₄-alkyl)formamids oder Di(C₁-C₄-alkyl)acetamids oder in N-Methyl-2-pyrrolidon durchgeführt wird und Palladium als Katalysator verwendet wird.

7. Verfahren nach einem der Ansprüche 1 bis 6, umfassend zusätzlich die anschließende Umwandlung von Diolen (Ia) und (Ib) in die entsprechenden Verbindungen (IVa) und (IVb) 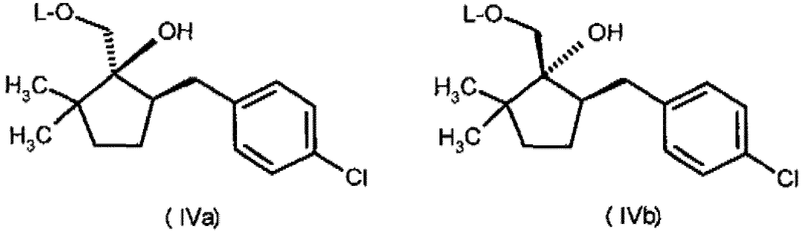 wobei L für C₁-C₆-Alkylsulfonyl, C₁-C₆-Halogenalkylsulfonyl, Phenylsulfonyl oder durch Halogen, C₁-C₄-Alkyl oder Nitro substituiertes Phenylsulfonyl steht, und entweder
a) die anschließende Umsetzung von (IVa) und (IVb) mit einem Triazol (V) 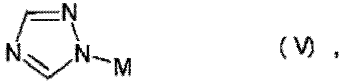 wobei M für ein Alkalimetall steht, in Gegenwart einer Base oder
b) die Umwandlung von (IVa) und (IVb) in Gegenwart einer Base in die Epoxide (VIa) und (VIb) 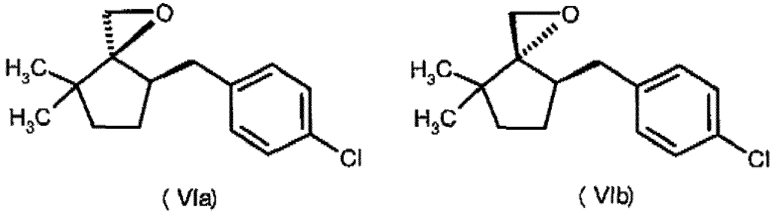 und die anschließende Umsetzung von (VIa) und (VIb) mit einem Triazol (V) zu Metconazol (Xa) / (Xb)

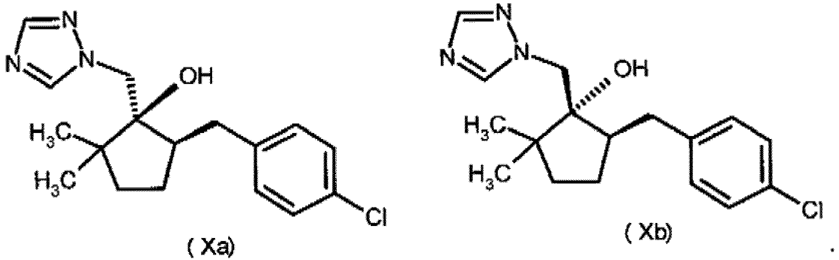

8. 5-[1-(4-Chlorphenyl)meth-(E)-yliden]-1-hydroxy-methyl-2,2-dimethylcyclopentanol der Formel (III)

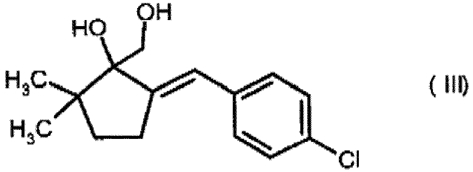

## Revendications

1. Procédé de préparation de diols de la formule (Ia) et (Ib) 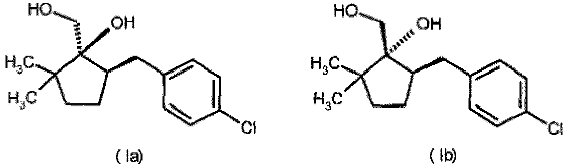 comprenant a) la réaction d'un oxirane (II) 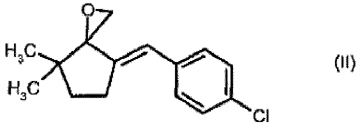 avec de l'eau et b) l'hydrogénation du 5-[1-(4-chlorophényl)-méth-(E)-ylidène]-1-hydroxyméthyl-2,2-diméthyl-cyclopentanol (III) résultant 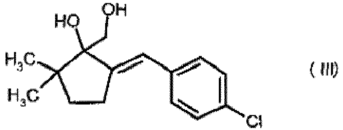 avec de l'hydrogène en présence d'un catalyseur métallique.

2. Procédé selon la revendication 1, dans lequel la réaction a) est réalisée à une température de -10 à 40°C.

3. Procédé selon la revendication 1, dans lequel l'hydrogénation b) est réalisée à une température de -10 à 40°C.

4. Procédé selon les revendications 1 à 3, dans lequel la réaction est réalisée dans un solvant ou un mélange de solvants.

5. Procédé selon la revendication 4, dans lequel le catalyseur, la température et le solvant ou mélange de solvants sont choisis de telle sorte que le diol (Ia) est obtenu en excès par rapport au diol (Ib).

6. Procédé selon la revendication 4, dans lequel l'hydrogénation est réalisée en présence d'un di(alkyl en C₁-C₄) formamide, d'un di (alkyl en C₁-C₄)acétamide ou dans de la N-méthyl-2-pyrrolidone et du palladium est utilisé comme catalyseur.

7. Procédé selon les revendications 1 à 6, comprenant également la transformation consécutive des diols (Ia) et (Ib) en composés correspondants (IVa) et (IVb) 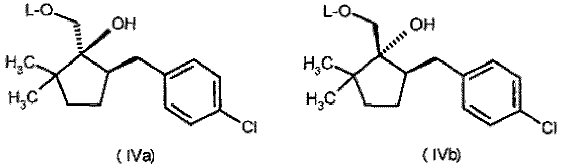 dans lesquels L est un radical alkylsulfonyle en C₁-C₆, halogénoalkylsulfonyle en C₁-C₆, phénylsulfonyle ou phénylsulfonyle substitué par un halogène, un alkyle en C₁-C₄ ou un groupe nitro, et
a) la réaction consécutive de (IVa) et (IVb) avec un triazole (V) 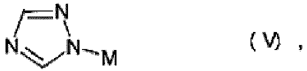 dans lequel M est un métal alcalin, en présence d'une base, ou
b) la transformation de (IVa) et (IVb) en présence d'une base en époxydes (VIa) et (VIb) 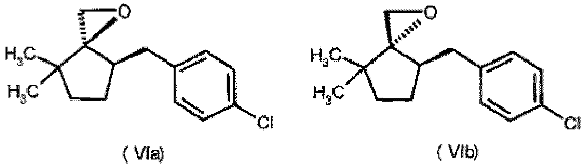 et la réaction consécutive de (VIa) et (VIb) avec un triazole (V), pour obtenir du metconazole (Xa) / (Xb)

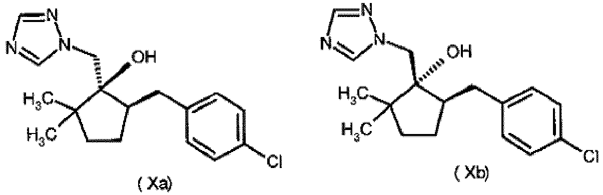

8. 5-[1-(4-Chlorophényl)-méth-(E)-ylidène]-1-hydroxyméthyl-2,2-diméthyl-cyclopentanol de la formule (III)
